# EUROPEAN PATENT APPLICATION

(11) **EP 3 925 639 A1**
(43) Date of publication of application: **22.12.2021**
(21) Application number: 21179675.0
(22) Date of filing: 16.06.2021
(51) Int. Cl.: A61M 1/00

(54) **CLOSURE LID FOR COLLECTION JARS IN SUCTION SYSTEMS, RELATED COLLECTION JAR AND RELATED SUCTION SYSTEM**

(30) Priority: 17.06.2020 IT 202000014476
(71) Applicant: Flaem Nuova S.p.A., 25015 Desenzano del Garda (BS) (IT)
(72) Inventor: ABATE, Riccardo, 25015 Desenzano del Garda (BS) (IT); ONGARETTI, Luca, 25060 Cellatica (BS) (IT); VENTURI, Alessandro, 25015 Desenzano del Garda (BS) (IT)
(74) Representative: Botti, Mario

(57) **Abstract**

The present invention relates to a closure lid (1) for collection jars (100) in suction systems comprising an internal portion (12) made of a first material, a gasket portion (13) that is co-molded on at least one perimeter sector (14) of the internal portion (12), the gasket portion (13) being adapted to be interposed between the closure lid (1) and a container (2) of a collection jar (100) to removably seal-couple the closure lid (1) on the container (2), the gasket portion (13) being made of a second material, at least one fluid inlet (4), at least one fluid outlet (5) at the gasket portion (13) and at least one seat for a filter (16, 16') integrally made at the at least one fluid outlet (5).

The present invention also relates to a related collection jar and a related suction system.

## Description

### Field of application

The present invention relates to a closure lid for collection jars in suction systems.

The present invention also relates to a related collection jar and to a related suction system.

Specifically, this invention finds a possible, but not exclusive, application in the healthcare field in medical suction operations, mainly in designated structures such as hospitals and nursing homes.

### Prior art

As it is well known in this specific technical filed, surgical aspirators are used during and after surgery to remove both surgical and body fluids, including tissues or even small bone fragments.

Surgical aspirators normally include a pump or a vacuum source adapted to perform the active suction function and collection elements adapted to contain the fluids and tissues sucked during the operation, also called "collection jars".

Said collection jars may be of the "disposable" type, namely used for a single patient, or of the "reusable" type, namely sterilizable and usable for multiple patients.

The collection jars generally comprise a disposable collection bag contained within a rigid containment element. A lid having openings for receiving and expelling sucked fluids and tissues is therefore provided.

This lid is basically made of plastic material and is placed on top of the rigid element, usually made of transparent polycarbonate. A gasket interposed between the container and the lid is often further adopted to allow a seal coupling, generally made of silicone.

This coupling is not always optimal, since it is necessary to have a correct centering of the components. A quick assembly action, such as that often required for these components due to their function, may jeopardize said centering.

Furthermore, a purification filter is connected at an outlet of the collection jar by means of a connection tube to protect the suction pump from any contamination.

This series of components connected in series, i.e. the collection jar, the connection tube, and the filter, although suitable for the function in charge, are impractical and subjected to possible disconnections and consequent malfunctions of the entire system, which can affect the timing of the operations and, in some cases, the success thereof.

WO 2014/089056 A1 patent application attempts to remedy this drawback with a lid frictionally coupled to a collection container, said lid accommodating a replaceable filtering cartridge containing a filter element, and into which a ball float is further engaged.

Although this solution is advantageous in some aspects, it is particularly limited in its applicability and particularly complex in its implementation.

The technical problem underlying the present invention is to devise a collection jar that ensures at the same time both a high functionality of the jar and of the system in which it is engaged, and greater practicality and versatility of use, overcoming the limits of the solutions currently proposed by the prior art.

Another purpose is to provide a collection jar that is adaptable to a plurality of pre-existing systems without the need for special reconfigurations.

A further purpose is to provide a collection jar that also allows a reduction of the adopted components and a better functional coupling therebetween.

Finally, a further purpose is to provide an optimized system both in relation to production methods and times and in relation to feasibility costs.

### Summary of the invention

The solution idea underling the present invention is to provide a lid for the collection jar having portions with specific features both in terms of coupling between the components and in terms of operation of the collection jar.

The above technical problem is solved by a closure lid for collection jars in suction systems comprising an internal portion made of a first material, a gasket portion that is co-molded on at least one perimeter sector of the internal portion, the gasket portion being adapted to be interposed between the closure lid and a container of a collection jar to removably seal-couple the closure lid on the container, the gasket portion being made of a second material, at least one fluid inlet, at least one fluid outlet at said gasket portion and at least one seat for a filter that is integrally made at the at least one fluid outlet.

Advantageously, the solution according to the present invention allows obtaining excellent operating features from the sealing point of view, eliminating assembly phases, and also giving strength and durability to the component.

Moreover, advantageously there is a reduction in the number of components by means of an ad hoc coupling therebetween.

Preferably, the first material is a thermoplastic material selected from PP, PC, POM, PE, HDPE, and the second material is an elastomeric material selected from TPE, TPU, silicone.

Advantageously, these materials are both reliable from a functional point of view and widely available.

More preferably, the first material is PP, and the second material is TPE.

Advantageously, these materials are the most similar for reciprocal co-molding and the most suitable for carrying out the required functions.

Preferably, the at least one fluid inlet is also made at the gasket portion on the internal portion.

Advantageously, this solution is optimal for sealing even at the connection to the suction of the closure lid.

According to another aspect, the present invention provides a collection jar for suction systems comprising a container and at least one lid according to the above-mentioned specifications.

Advantageously, the solution according to the present invention allows ensuring the coupling stability of the collection lid on the container, in addition to the above reduction of the involved components and to the improvement of the production process.

According to a preferred embodiment, the collection jar further comprises a filter that is directly seal-engaged in the seat for filter.

Advantageously the present solution ensures excellent performance with a functional optimization with a perfect sealing, a productive optimization with a streamlining of the necessary phases, high applicability in pre-existing systems, a saving in weight and components and therefore also a saving in the production costs.

According to an alternative embodiment, the collection jar further comprises a filter engaged in the seat for filter and a block and separation cap that is adapted to keep the filter in position in the seat for filter and comprising an outlet portion that is functionally connected to the at least one outlet of the closure lid.

Advantageously, this solution is highly performing, and the lid not only allows keeping the filter in position, but also as a barrier for any external fluids.

More preferably, according to the latter embodiment, the filter is made of highly breathable and porous material with a very open cell.

Advantageously, this solution is ideal for hydrophobic, antibacterial, and antiviral applications.

Still preferably, the collection jar further comprises a suction cannula at the at least one fluid inlet and at least one internal float safety device in the container.

Advantageously, this feature allows stopping the suction above the maximum liquid level provided by the collection jar.

According to a further aspect, the present invention provides a suction system comprising at least one suction pump and at least one collection jar having the above features.

Advantageously, a suction system thus realized is optimized and functional starting from the production process up to the use thereof.

Further features and advantages will become clearer from the following detailed description of a preferred, but not exclusive, embodiment, of the present invention, with reference to the enclosed figures given by way of non-limiting example.

### Brief description of the drawings

In these drawings:
Figure 1 shows a perspective view of a first embodiment according to the invention;
Figure 2 shows an exploded view of the embodiment of Figure 1;
Figure 3A shows a sectional view of the embodiment of Figure 1;
Figures 3B and 3C show two further sectional views of details of the embodiment of Figure 1;
Figure 4 shows a perspective view of a second embodiment according to the invention;
Figure 5 shows an exploded view of the embodiment of Figure 4;
Figure 6A shows a sectional view of the embodiment of Figure 4;
Figures 6B and 6C show two further sectional views of details of the embodiment of Figure 4.

In the different figures, analogous elements will be identified by analogous reference numbers.

### Detailed description

With reference to these figures, reference number 100 globally and schematically indicates a collection jar according to the present invention.

The collection jar 100 comprises a closure lid 1 and a container 2. The closure lid is removably seal-coupled on the container 2.

At least one collection jar 100 is engaged on a fluid path in a suction system (not shown), which further comprises a suction pump, or a vacuum source, that is functionally coupled to said least one collection jar 100.

The collection jar 100 further comprises a filter 3, 3' that is coupled to the hygroscopic, antibacterial and antiviral closure lid 1 to avoid contamination of the suction system wherein it is engaged.

The closure lid 1 comprises at least one fluid inlet 4 and at least one fluid outlet 5.

The fluid inlet 4 is made of a through-tube that is integrally made in the closure lid 1, projecting outward and inward from the closure lid 1.

At an end of the fluid inlet 4 facing towards the inside of the collection jar 100 there is a hook section for directing the inlet fluid.

At the inlet 4 a suction cannula 6 is connected, which puts in communication the collection jar 1 with the patient, preferably by means of a silicone tube (not shown).

The fluid outlet 5 is also made of a through-tube that is integrally made in the closure lid 1, projecting outward and inward from the closure lid 1.

On the inward-projecting portion 5A the fluid outlet 5 provides a plurality of reinforcement fins 17 arranged in a radial pattern to give greater strength and structural rigidity to the fluid outlet 5 itself.

Furthermore, on the inward-projecting portion 5A, in the represented preferred embodiments, at least one float security device 7 is coupled, which is adapted to stop the fluid suction inside the collection jar 100 once a predetermined maximum threshold level has been reached.

Specifically, the float security device 7, in the represented preferred embodiments, provides an engagement cover 7A that is partially inserted on the inward-projecting portion 5A. On the internal surface of the engagement cover 7A there is provided at least one abutment surface 8 that is adapted to limit the insertion on the inward-projecting portion 5A.

At the bottom surface 9 of the engagement cover 7A a hole 10 is provided.

Inside the hollow space of the engagement cover 7A a float 7B is inserted. The float 7B is placed at the inward-projecting portion 5A and provides a closure valve 11 at the end facing toward said inward-projecting portion 5A.

When the fluid inside the collection jar 100 reaches the bottom surface 9 of the engagement cover 7A and starts entering through the hole 10, the fluid thus starts raising the float 7B. Once the float 7B causes the closure valve 11 to obturate the inward-projecting portion 5A, the maximum threshold level has been reached and the suction operation stops.

Nothing prevents from providing a different solution, depending on specific applications or particular production needs, all variants being included in the scope defined in the appended claims.

The closure lid 1 provides an internal portion 12 and a gasket portion 13.

The gasket portion further provides a closure element 21 of the fluid communication channels that is integrally made with the gasket, useful to close the container full of secretion after the use and/or for transporting the jar. These lids have a shape and diameters such as to ensure the sealing of the system and avoid the leakage of liquids.

In the embodiments represented by way of example in the Figures two closure elements 21 are preferably adopted.

The gasket portion 13 is co-molded on at least one perimeter sector 14 of the internal portion 12.

In this way, functional optimization is achieved since a perfect sealing of the closure lid 1 is ensured on the container 2, which is more efficient than the two-component configuration used in prior art devices.

Furthermore, this allows a streamlining of the necessary phases within the production process, which follows a single workflow without the need of using parallel flows with subsequent couplings that are more prone to errors.

Preferably, the internal portion 12 provides a tapered conformation towards the perimeter sector 14 in order to maximize the possible sealing surface with the co-molded gasket portion 13. Specifically, at the tapers the internal portion 12 provides gripping teeth 15 that are inserted by positively fitting in the gasket portion 13. Such a conformation ensures greater sealing and greater resistance to wear during use.

In a preferred embodiment the internal portion 12 is made of PP. In fact, this material has excellent structural features, being at the same time light, resistant to heat, humidity, contact with solvents and acids, and resistant to wear. Furthermore, PP is ideal for the preferred field of application of the present invention since it is non-toxic and suitable for sterilization procedures.

However, nothing prevents the adoption of different thermoplastic materials suitable for the internal portion 12 such as PC, POM, PE, and HDPE.

The gasket portion 13 is instead preferably made of TPE. In fact, this material has excellent features that are functional to its operating phase, in particular related to resistance and flexibility. Furthermore, TPE is particularly suitable in the production phase, being more processable and having lower density and therefore reducing the amount of material needed for the same volume.

However, nothing prevents the use of different elastomeric materials such as, for example, TPU or silicone.

Furthermore, the fluid outlet 5 is made at the gasket portion 13 that is co-molded on the internal portion 12, preferably by overmoulding in the same material.

This allows a perfect sealing of the connected components.

In a preferred embodiment, the fluid inlet 4 is also made at the gasket portion 13 that is co-molded on the internal portion 12.

This solution is optimal for ensuring the sealing between the components even at the suction, thus avoiding problems during the operating phase of the system which the collection jar is connected to.

Still preferably, the gasket portion 13 has two opposite semicircular expansions 13A, 13B, which develop from the perimeter towards the center of the closure lid 1.

According to the present alternative, the fluid inlet 4 and the fluid outlet 5 are each arranged in one of the two semicircular expansions 13A, 13B.

According to the present invention, a seat for filter 16, 16', which is integrally made on the closure lid 1 at the at least one fluid outlet 5, is further provided.

Said seat for filter 16, 16' is intended to accommodate the above filter 3. 3'.

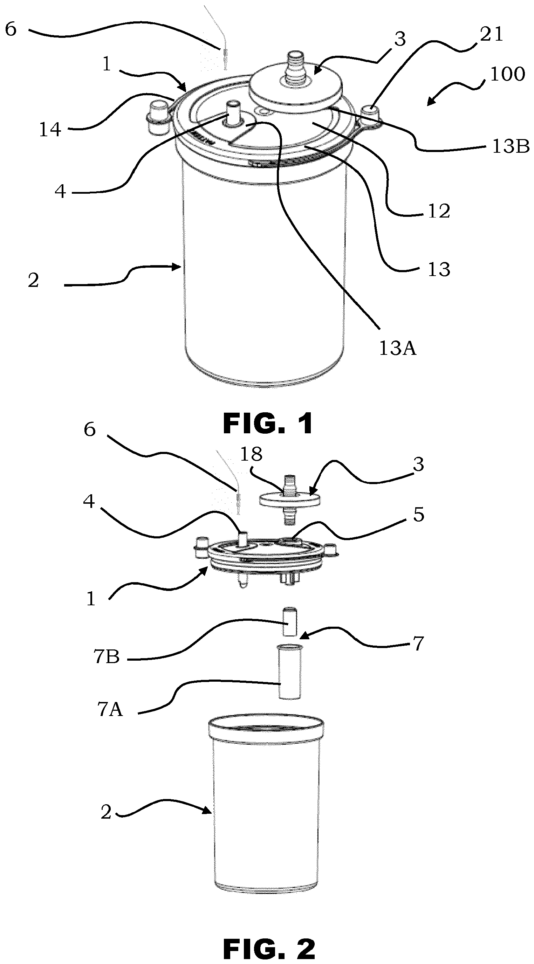

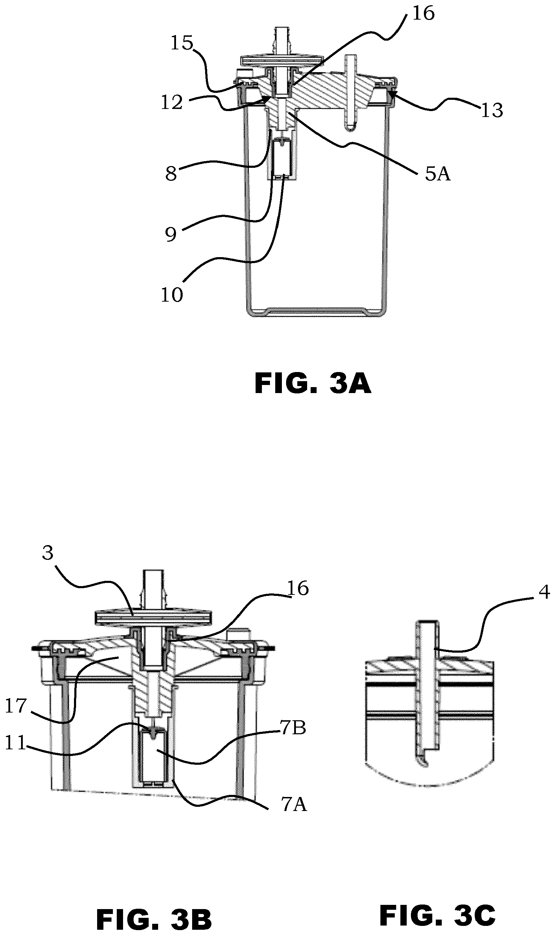

According to a first preferred embodiment, specifically shown in Figures 1 to 3C, the closure lid 1 provides a seat for filter 16 that is shaped so as to allow direct engagement of the filter 3 into the closure lid 1 with a seal-coupling, thanks to the presence of the gasket portion 13 that is co-molded at the fluid outlet 5.

The provided conformation is preferably and substantially of the inverted bottle neck type, slightly projecting from an external surface 17 of the closure lid 1. The seat for filter 16 provides a maximum size such as to also act as abutment for an external portion 18 of a filter 3 of the collection jar 100. In this way, the engagement of the filter 3 into the seat for filter 16 is facilitated and safe, since it does not require forcing in the initial phase and only stops under a perfectly sealing condition, thanks to the friction generated by the gasket portion 13 and thanks to the abutment function of the seat for filter 16 on said external portion 18 of the filter 3.

The hooking mechanism herein defined between the filter 3 and the seat for filter 16 ensures excellent performance in the fluid outlet phase from the collection jar 100 but also with a saving in weight.

Furthermore, this solution is particularly suitable for conforming to the currently most adopted filters, thus not requiring difficult reconfigurations of the pre-existing systems.

Advantageously, moreover, such a conformed solution allows a high applicability in pre-existing systems, a saving in weight and components and consequently also a saving in the production costs.

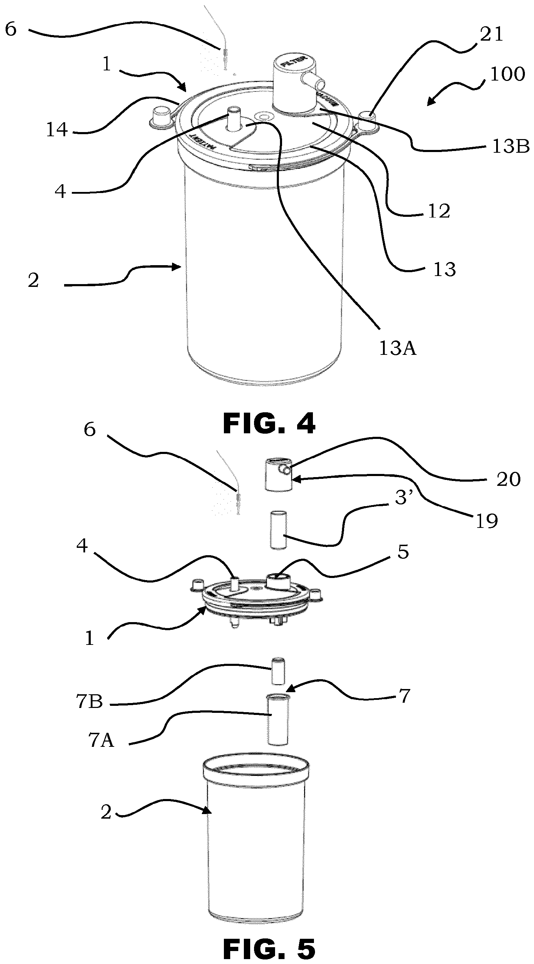

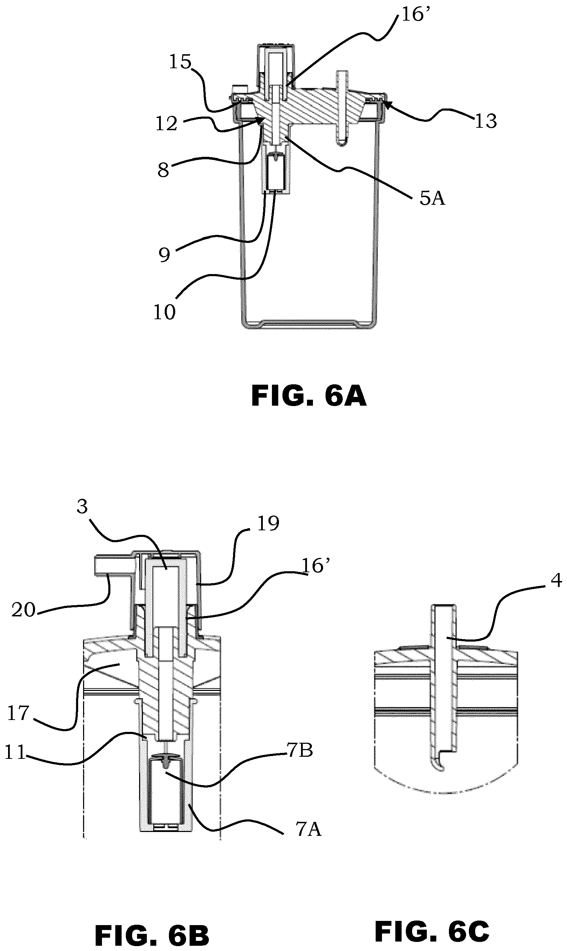

According to an alternative embodiment, particularly shown in Figures 4 to 6C, the collection jar 100 comprises a filter 3' engaged in a seat for filter 16'.

The integral seat for filter 16' has a substantially cylindrical conformation. The adopted filter 3' is preferably made of a highly breathable and porous material with a very open cell, still with hydrophobic, antibacterial and antiviral features.

The filter 3' comprises a block and separation cap 19 that is adapted to keep the filter 3 in position in the seat for filter 16'.

The block and separation cap 19 further comprises an outlet portion 20 that is functionally connected to the at least one outlet 5 of the closure lid 1.

La seat per filter 16' is slightly projecting so as to maximize the space available to accommodate the filter 3'. The block and separation cap 19 is therefore placed in a hood overlapping the seat for filter 16' and is coupled there by friction between its internal surface and the external surface of the seat for filter 16'.

The outlet portion 20 in the represented embodiment is perpendicular to the development of the filter 3' and parallel to the closure lid 1 to facilitate the use phase.

Advantageously, the present invention allows obtaining excellent and lasting sealing performance under all conditions of use.

Further advantageously, the present invention allows an optimization of the production cycle, with a reduction of production errors and component coupling and with a consequent impact also on the production times and related costs.

Furthermore, advantageously, the present invention is of practical use by operators who often work under conditions of maximum required execution speed.

The skilled person will also appreciate how the present invention is widely applicable within pre-existing systems without the need for complex reconfigurations.

The skilled person will appreciate that the embodiments of the presented lid, of the related collection jar and of the suction system may be subjected to various changes and variations, according to specific and contingent needs, all within the scope of protection of the invention, as defined by the following claims.

For instance, it is possible to make changes to the adopted materials that go hand in hand with their evolution over time or to provide for a different arrangement of the components.

## Claims

1. Closure lid (1) for collection jars (100) in suction systems comprising:
- an internal portion (12) made of a first material;
- a gasket portion (13) that is co-molded on at least one perimeter sector (14) of said internal portion (12), said gasket portion (13) being adapted to be interposed between said closure lid (1) and a container (2) of a collection jar (100) to removably seal-couple said closure lid (1) on said container (2), said gasket portion (13) being made of a second material;
- at least one fluid inlet (4);
- at least one fluid outlet (5) at said gasket portion (13);
- at least one seat for a filter (16, 16') integrally made at said at least one fluid outlet (5).

2. Closure lid (1) according to claim 1, wherein said first material is a thermoplastic material selected among PP, PC, POM, PE, HDPE, and wherein said second material is an elastomeric material selected among TPE, TPU, silicon.

3. Closure lid (1) according to claim 2, wherein said first material is PP and wherein said second material is TPE.

4. Closure lid (1) according to any one of claims 1 to 3, wherein said at least one fluid inlet (4) is made at said gasket portion (13) on said internal portion.

5. Collection jar (100) for suction systems comprising a container (2) and at least one closure lid (1) according to any one of claims 1 to 4.

6. Collection jar (100) according to claim 5, further comprising a filter (3) that is directly seal-engaged in said seat for filter (16).

7. Collection jar (100) according to claim 5, further comprising a filter (3') engaged in said seat for filter (16') and a block and separation cap (19) that is adapted to keep said filter (3') in position in said seat for filter (16') and comprising an outlet portion (20) that is functionally connected to said at least one fluid outlet (5) of said closure lid (1).

8. Collection jar (100) according to claim 7, wherein said filter (3') is made of highly breathable and porous material with a very open cell.

9. Collection jar (100) according to any one of claims 5 to 8, further comprising a suction cannula (6) at said at least one fluid inlet (4) and at least one internal float safety device in said container (2).

10. Suction system comprising at least one suction pump and at least one collection jar (100) according to any one of claims 5 to 9.
